# EUROPÄISCHE PATENTSCHRIFT

(11) **EP 0 592 933 B1**
(45) Veröffentlichungstag und Bekanntmachung des Hinweises auf die Patenterteilung: **04.09.1996**
(21) Anmeldenummer: 93116144.2
(22) Anmeldetag: 06.10.1993
(51) Int. Cl.: C07C 327/22, C08F 20/38

(54) **Verfahren zur Herstellung von Thiol(meth)acrylaten**
Method for the preparation of thiol(meth)acrylates
Procédé pour la préparation des thio(meth)acrylates

(30) Priorität: 10.10.1992 DE 4234257
(43) Veröffentlichungstag der Anmeldung: 20.04.1994
(73) Patentinhaber: RÖHM GMBH, D-64293 Darmstadt (DE)
(72) Erfinder: Bader, Martina, Dr., D-64347 Griesheim (DE); Hartmann, Patrik, D-64572 Büttelborn (DE); Schwinn, Gerhard, D-64807 Dieburg (DE)

(56) Entgegenhaltungen:
- PATENT ABSTRACTS OF JAPAN vol. 16, no. 71 (C-913)21. Februar 1992 & JP-A-03 264 549 (SANYO CHEMICAL INDUSTRY) 25. November 1991
- H. Rauch-Puntigam, Th. Völker, Acryl.- und Methacrylverbindungen S. 36, 64, Springer Verlag 1967

## Beschreibung

Die Erfindung betrifft ein Verfahren zur Herstellung von Thiol(meth)acrylaten, ausgehend von (Meth)acrylsäureanhydrid.

### Stand der Technik

Die Darstellung von Estern der Thiocarbonsäuren, die selbst als "aktive Ester" einzustufen sind, kann unter dem Gesichtspunkt zusammengefaßt werden, daß dazu von reaktiven Carbonsäurederivaten auszugehen ist. So ist beispielsweise die Herstellung von Thiol(meth)acrylsäureestern aus den entsprechenden Carbonsäurehalogeniden bis dato die Methode der Wahl (vgl. M.M. Koton et al. Zhur.Obshchei Khim. 26, 475-6 (1956); Chem. Abstr. 50, 13815; JP 02 229 808; JP 03 11 054). Die Synthese von Dithioldi(meth)acrylaten aus 3-Chlor-(2-methyl)propionsäurechlorid und Dithiolen ist Gegenstand des Japan. Patents 02 172 969. In JP 02 003 675 wird die Herstellung von Thiol(meth)acrylaten aus dem (Meth)acrylchlorid unter Phasentransferkatalyse beschrieben. Mit der gleichen Methodik sind auch Dithioldimethacrylate, wie in EP-A 273 661 beschrieben, zugänglich.

In der JP-A 03 264 549 wird ein Herstellungsverfahren für hochreine Methacryloyl-Monomere durch Umsetzung von Methacrylsäureanhydrid mit Alkoholen inclusive Polyolen. Thiolen u. Aminen vorgeschlagen, wobei das erfinderische Element in der Temperaturkontrolle, d.h. in der Durchführung der Umsetzung bei höchstens 15°C gesehen wird.

### Aufgabe und Lösung

Die Umsetzung mit den Säurehalogeniden der (Meth)acrylsäure wirft eine Reihe von Problemen auf. So ist z.B. Methacrylsäurechlorid eine stechend riechende, bei Luftzutritt rauchende, die Augen stark reizende Substanz, die selbst Polymerisationstendenz zeigt. Das leicht flüchtige, stark schleimhautreizende Acrylchlorid polymerisiert bereits im Licht. (Vgl. H. Rauch-Puntigam, Th. Völker Acryl- und Methacrylverbindungen, Springer-Verlag Berlin, Heidelberg, New York 1967, S. 19, 43). Es stellte sich daher die Aufgabe, ein Verfahren zur Herstellung von Estern der Methacrylsäure von sowohl aromatischen als aliphatischen Alkoholen zu finden, das nicht von den Säurehalogeniden ausgeht. Andererseits wächst mit abnehmender Aktivierung der Carbonylfunktion die Wahrscheinlichkeit der Reaktion an der Doppelbindung z.B. durch Addition von Nucleophilen oder Additionspolymerisation.
Bei einer Umsetzung von Thiolen mit (Meth)acrylsäurederivaten, die eine geringere Carbonylaktivität als die der Säurehalogende besitzen, war z.B. mit einer Addition des Thiols an die aktivierte Doppelbindung zur rechnen. (Vgl. Houben-Weyl, IV. Aufl., Band IX, S. 124 - 125, Georg Thieme-Verlag 1955). Moleküle mit zwei (aktivierten) Doppelbindungen im Molekül wie z.B. Methacrylsäureanhydrid bieten eine erhöhte Additionsmöglichkeit, so daß eine einigermassen problemlose Reaktion zum Thioester nicht erwartet werden konnte, insbesondere bei Temperaturen oberhalb Raumtemperatur Entgegen den Erwartungen wurde nun gefunden, daß Thio(meth)acrylate durch Umsetzung eines Thiols mit (Meth)acrylsäureanhydrid herstellbar sind, wobei die Reaktion hauptsächlich oberhalb Raumtemperatur stattfindet.

Die Erfindung betrifft somit ein Verfahren zur Herstellung der Thiol(meth)acrylsäureester der allgemeinen Formel I 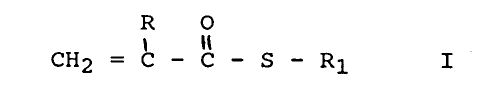 worin
- R: für Wasserstoff oder Methyl und
- R₁: für einen gegebenenfalls substituierten Phenylrest oder für einen gegebenenfalls substituierten, gegebenenfalls cyclischen Alkylrest mit 1 bis 24, vorzugsweise 1 bis 12 Kohlenstoffatomen steht
wobei man (Meth)acrylsäureanhydrid der Formel II 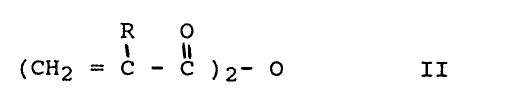 worin
- R: für Wasserstoff oder Methyl steht,
in mindestens stöchiometrischer Menge mit einem Thiol oder Thiolat der Formel III

R₁ - S - M III

worin
- M: für Wasserstoff oder ein Metall, insbesondere ein Alkalikation steht und
- R₁: die oben bezeichnete Bedeutung besitzt, in wässrig-alkalischer Lösung versetzt und anschliessend bei Temperaturen oberhalb Raumtemperatur
umsetzt. Vorzugsweise führt man die Reaktion so durch, daß das Anhydrid der Formel II in einem geeigneten, inerten, in der Regel nicht mit Wasser mischbaren organischen Lösungsmittel L zur Anwendung kommt.

Die Reaktion wird in Anwesenheit von Base durchgeführt, insbesondere zu beachten wenn das Thiol in freier Form (M = Wasserstoff) eingesetzt wird. Als Base ist Alkalihydroxid oder Alkalicarbonat bevorzugt, insbesondere mit Natrium oder Kalium als Kationen. Auch wenn M für ein Metallkation steht, sind Natrium und Kalium bevorzugt.

### Durchführung der Reaktion

Die Herstellung der Ausgangsverbindung der Formel II, des (Meth)acrylsäureanhydrids ist seit langem bekannt (vgl. H. Rauch-Puntigam, Th. Völker, Acryl- und Methacrylverbindungen loc.cit., Seite 19, 20, 43, 44). Als Säureanhydride mit zusätzlicher Aktivierung der Doppelbindung zersetzen sich diese Flüssigkeiten mit Wasser und zeigen ausgeprägte Polymerisationsneigung: Eigenschaften, die bei der Handhabung zu beachten sind, ebenso wie die Reizung der Schleimhäute durch die Dämpfe. Die Thiole der Formel III sind ebenfalls bekannt (vgl. Houben-Weyl, Methoden der organischen Chemie, IV. Auflage, Bd. IX, lo.cit. S. 3 - 48). Besonders genannt seien Alkylthiole wie Propanthiol, Butanthiol, Pentanthiol, Hexanthiol, Heptanthiol, Octanthiol, Nonanthiol, Dekanthiol, Dodekanthiol, ferner (Thio)ethermercaptane wie z.B. 2,2'-Dithiodiethylether, 2,2'-Dimercaptoethylsulfid, substituierte Alkylthiole wie Ethyl-3-mercaptopropionat, Ethyl-2-mercaptoacetat oder aromatische Verbindungen wie Phenylethanthiol, Thiophenol. Soweit es sich um substituierte Reste R₁ handelt, so sind dies vorzugsweise Carboxylgruppen, die mit C₁-C₆-Alkoholen verestert sein können, C₁-C₄-Alkyl oder Halogen, insbesondere Fluor, Chlor und Brom. Der Umsatz zwischen den Verbindungen II und III erfolgt im stöchiometrischen Verhältnis 1 : 1, es ist aber vorteilhaft, das Säureanhydrid II in einem gewissen Überschuß einzusetzen, beispielsweise im 0,05 bis 0,5-fachen Überschuß über die stöchiometrisch erforderliche Menge an III.
Als inertes Lösungsmittel L, in dem die Verbindung II vorzugsweise zur Anwendung kommt, eignen sich z.B. aromatische Kohlenwasserstoffe wie Toluol, Xylol oder Ether wie Methyl-tert.Butylether (MTBE). Auch empfiehlt sich die Stabilisierung des Anhydrids II durch Zusatz eines Polymerisations-Inhibitors z.B. aus der Klasse der sterisch gehinderten Phenole, z.B. mit 4-Methyl-2,6-ditert.butylphenol, 2,4-Dimethyl-6-tert.butylphenol, tert.Butylbenzkatechin, der chinoiden Verbindungen wie Hydrochinonmonomethylether u.ä., im allgemeinen in Mengen zwischen 0,01 und 0,2 Gew.-% bezogen auf die polymerisationsfähige Substanz.

Die Reaktion kann z.B. in folgender Weise durchgeführt werden:
In einem mit Rührer versehenen Reaktionsgefäß gibt man das Anhydrid der Formel II im Lösungsmittel L vor. Als Anhalt sei etwa die 5 - 10-fache Menge an Lösungsmittel bezogen auf das Gewicht von II genannt. Vorzugsweise kühlt man mit Eiskühlung und tropft das Thiol III, beispielsweise in etwa der vierfachen Gewichtsmenge einer wäßrigen Alkalilauge - genannt sei ca. 10 %-ige Natronlauge - über einen gewissen Zeitraum, z.B. ca. 60 Minuten zu. Im allgemeinen wird ein pH-Bereich von ca. 7 bis 10 eingehalten. Man rührt noch einige Zeit, beispielsweise ca. 3 Stunden, bei Temperaturen über Raumtemperatur, beispielsweise bei 40 Grad C nach. Anschließend wird die organische Phase über einen Trichter abgetrennt und einmal mit Wasser bzw. einer wäßrigen, alkalischen Lösung, beispielsweise vom pH 12 zur Entfernung gebildeter (Meth)acrylsäure bzw. mit Wasser gewaschen. Man trocknet zweckmäßig über einem geeigneten Trockenmittel wie z.B. Natriumsulfat entfernt das Lösungsmittel, z.B. im Rotationsverdampfer. Sofern das gewonnene Rohprodukt noch nicht den Anforderungen entspricht, gewinnt man zweckmäßigerweise daraus das Reinprodukt der Formel I durch Destillation im Hochvakuum. Mitunter enthalten die Rohprodukte einen geringen Anteil an Additionsprodukt aus Thiol und Thioester, das sich aber destillativ entfernen läßt.

### Vorteilhafte Eigenschaften

Die erfindungsgemäß hergestellten Monomeren lassen sich beispielsweise zu transparenten, hochlichtbrechenden Kunststoffen verarbeiten.
Derartige Kunststoffe lassen sich zu einer Vielfalt optischer Gerätschaften und Artikel verarbeiten, beispielsweise zu Linsen für Brillenglasmaterial usw.

Die folgenden Beispiele dienen zur Erläuterung der Erfindung.

### BEISPIELE

### Beispiel 1

### Herstellung von Hexylthiomethacrylat

Zu einer Lösung von 49,9 g Methacrylsäureanhydrid (stabilisiert mit 1 000 ppm 4-Methyl-2,6-ditert.butylphenol) in 450 ml Methyl-tert.butylether (MTBE) wird unter Eiskühlung eine Lösung von 35 g Hexanthiol in 140 ml 10 %iger Natronlauge zugetropft. Es wird 3 Stunden bei 40 Grad C nachgerührt. Die organische Phase wird abgetrennt und mit Wasser gewaschen. Nach Trocknung mit Natriumsulfat und Entfernen des Lösemittels erhält man nach Destillation das Hexylthiomethacrylat (Kp. 65 Grad C/ 0,3 mbar). Ausbeute: 83 % d.Th.
Die Strukturbestätigung erfolgt jeweils durch NMR, GC-MS, und IR.

### Beispiel 2

### Herstellung von Phenylthiomethacrylat

Zu einer Lösung von 77 g Methacrylsäureanhydrid (stabilisiert mit 1 000 ppm 4-Methyl-2,6-ditert.butylphenol) in 400 ml Methyl-tert.butylether wird unter Eiskühlung eine Lösung von 55,1 g Thiophenol und 20,4 g NaOH in 80 ml Wasser zugegeben. Es wird 3 Stunden bei 40 Grad C nachgerührt. Die organische Phase wird abgetrennt und mit Wasser gewaschen. Die getrocknete Rohesterlösung wird destilliert (Kp 70 - 72 Grad C/ 0,5 mbar). Man erhält das Phenylthiomethacrylat (Reinheit nach GC > 98 %). Ausbeute: 80 % d.Th.

### Beispiel 3

### Herstellung von Methylthiomethacrylat (Thiomethacrylsäure-S-methylester)

In ein Gemisch von 84,7 g Methacrylsäureanhydrid, 480 ml Methyltert.butylether und 240 ml 10 %ige NaOH-Lösung sowie 85 mg 4-Methyl-2,6-ditert.butylphenol wird 25 g gasförmiges Methylmercaptan unter Eiskühlung eingleitet. Nach Zugabeende wird 4 Stunden bei 40 Grad C gerührt. Nach Abkühlung wird die organische Phase abgetrennt, mit Wasser gewaschen und mit Natriumsulfat getrocknet. Nach Destillation erhält man 21,4 g Methylthiomethacrylat (Kp 46 - 50 Grad C/13 mbar, 36 % d.Th.).

### Beispiel 4

### Herstellung von 3-Thiabutylthiomethacrylat (2-Thiomethylethylthiomethacrylat)

Zu einer Lösung von 116 g Methacrylsäureanhydrid (stab. mit 116 mg 4-Methyl-2,6-ditert.butylphenol) in 800 ml Methyltert.butylether wird unter Kühlung eine Lösung von 76 g 3-Thiabutylmercaptan in 500 ml 6 %ige NaOH-Lösung zugetropft. Nach Zugabeende wird 4 Stunden bei 40 Grad C gerührt. Die organische Phase wird abgetrennt, mit Wasser gewaschen und mit Natriumsulfat getrocknet. Nach Destillation erhält man 85 g 3-Thiabutylthiomethacrylat (Kp 62 - 66 Grad C/0,2 - 0,3 mbar, 69 % d.Th.)

## Patentansprüche

1. Verfahren zur Herstellung von Thio(meth)acrylsäureestern der allgemeinen Formel I 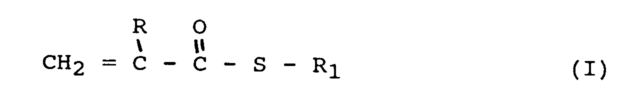 worin
R für Wasserstoff oder Methyl und
R₁ für einen gegebenenfalls substituierten Phenylrest oder für einen gegebenenfalls substituierten, gegebenenfalls cyclischen Alkylrest mit 1 bis 24 Kohlenstoffatomen steht,
dadurch gekennzeichnet,
daß man (Meth)acrylsäureanhydrid der Formel II 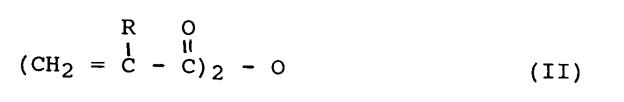 worin
R für Wasserstoff oder Methyl steht
mit einem Thiol oder Thiolat der Formel III
R₁ - S - M (III)
worin
M für Wasserstoff oder ein Metall-Kation steht und
R₁ die vorstehend bezeichneten Bedeutungen besitzt
in wäßrig-alkalischer Lösung versetzt und anschließend bei Temperaturen über Raumtemperatur umsetzt.

2. Verfahren gemäß Anspruch 1, dadurch gekennzeichnet, daß R₁ für einen Phenylrest oder einen Alkylrest mit 1 bis 6 Kohlenstoffatomen steht.

3. Verfahren gemäß den Ansprüchen 1 und 2, dadurch gekennzeichnet, daß M für Wasserstoff oder ein Alkalikation steht.

4. Verfahren gemäß Anspruch 3, dadurch gekennzeichnet, daß M für Natrium oder Kalium steht.

5. Verfahren gemäß den Ansprüchen 1 bis 4, dadurch gekennzeichnet, daß man das (Meth)acrylsäureanhydrid (II) im 0,05 bis 0,5-fachen stöchiometrischen Überschuß über die Verbindung der Formel (III) anwendet.

6. Verfahren gemäß den Ansprüchen 1 bis 5, dadurch gekennzeichnet, daß das (Meth)acrylsäureanhydrid der Formel II in einem inerten organischen, nicht mit Wasser mischbaren Lösungsmittel L zur Anwendung kommt.

## Claims

1. A process for preparing thio(meth)acrylic acid esters of general formula I 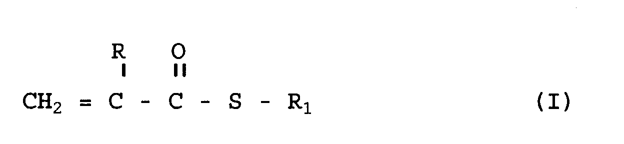 wherein
R is hydrogen or methyl and
R₁ is an optionally substituted phenyl group or an optionally substituted, optionally cyclic alkyl group having 1 to 24 carbon atoms,
characterised in that a (meth)acrylic acid anhydride of formula II 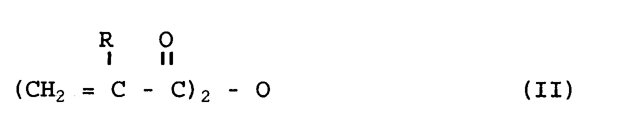 wherein
R is hydrogen or methyl
is mixed, in an aqueous alkaline solution with a thiol or thiolate of formula III
R₁ - S - M (III)
wherein
M is hydrogen or a metal cation and
R₁ has the above meanings,
and then reacted at temperatures above the ambient temperature.

2. A process according to Claim 1, characterised in that R₁ is a phenyl group or an alkyl group having 1 to 6 carbon atoms.

3. A process according to Claims 1 and 2, characterised in that M is hydrogen or an alkali metal cation.

4. A process according to Claim 3, characterised in that M is sodium or potassium.

5. A process according to Claims 1 to 4, characterised in that (meth)acrylic acid anhydride (II) is used in an excess of 0.05 to 0.5 times the stoichiometric equivalent of the compound of formula (III).

6. A process according to Claims 1 to 5, characterised in that the (meth)acrylic acid anhydride of formula (II) is used in an inert organic solvent L not miscible with water.

## Revendications

1. Procédé de préparation d'esters d'acide thio(méth)acrylique de formule générale 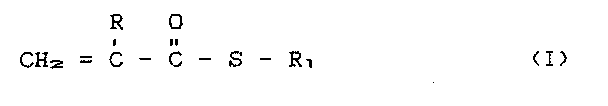 dans laquelle
R est mis pour un atome d'hydrogène ou un reste méthyle et
R₁ est mis pour un reste phényle éventuellement substitué ou pour un reste alkyle à 1-24 atomes de carbone éventuellement cyclique et éventuellement substitué,
caractérisé en ce que l'on mélange l'anhydride (méth)acrylique de formule II 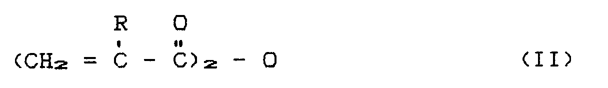 dans laquelle R est mis pour un atome d'hydrogène ou un reste méthyle,
avec un thiol ou un thiolate de formule III
R₁ - S - M (III)
dans laquelle
M est mis pour un atome d'hydrogène ou un cation de métal et
R₁ a les significations données ci-dessus,
en solution alcaline aqueuse, puis on les fait réagir à des températures supérieures à la température ambiante

2. Procédé selon la revendication 1, caractérisé en ce que R₁ est mis pour un reste phényle ou pour un reste alkyle à 1-6 atomes de carbone.

3. Procédé selon la revendication 1 ou 2, caractérisé en ce que M est mis pour un atome d'hydrogène ou pour un cation de métal alcalin.

4. Procédé selon la revendication 3, caractérisé en ce que M est mis pour un cation sodium ou potassium.

5. Procédé selon l'une quelconque des revendications 1 à 4, caractérisé en ce que l'on utilise l'anhydride (méth)acrylique (II) en excès stoechiométrique de 0,05 à 0,5 fois par rapport au composé de formule (III).

6. Procédé selon l'une quelconque des revendications 1 à 5, caractérisé en ce que l'anhydride (méth)acrylique de formule II est mis en oeuvre dans un solvant organique L inerte, non miscible à l'eau.
